# EUROPEAN PATENT APPLICATION

(11) **EP 1 640 017 A1**
(43) Date of publication of application: **29.03.2006**
(21) Application number: 04746432.6
(22) Date of filing: 18.06.2004
(51) Int. Cl.: A61K 38/16, A61P 25/00

(54) **REMEDIES FOR DISEASES WITH HYPERMYOTONIA**

(30) Priority: 20.06.2003 JP 2003175722; 06.11.2003 JP 2003377101
(71) Applicant: SANTEN PHARMACEUTICAL CO., LTD., Higashiyodogawa-ku, Osaka-shi, Osaka 533-8651 (JP)
(72) Inventor: KOZAKI, Shunji, Osaka 584-0071 (JP); SUGIMOTO, Nakaba, Hyogo 666-0257 (JP); SHIMAZAWA, M. Santen Pharmaceutical Co., Ltd., Nara 630-0101 (JP); HARA, Hideaki, Nara 630-8002 (JP)
(74) Representative: Peaucelle, Chantal
(86) International application number: PCT/JP2004/008963
(87) International publication number: WO 2004/112821

(57) **Abstract**

An M toxin of type A botulinum toxin (HA-negative substance) and a mixture of L toxin and LL toxin (HA-positive substance) are compared and examined in inhibitory action for neuromuscular transmission and therapeutic index. As a result, it is found that M toxin of type A botulinum toxin has characteristics of: 1) having an excellent inhibitory action for neuromuscular transmission; 2) showing a high therapeutic index; 3) showing a low antigenicity and 4) suffering from little reduction in efficacy even after repeatedly administered, compared with the
mixture of L toxin and LL toxin. Owing to these characterics, the M toxin of type A botulinum toxin is particularly useful as a therapeutic agent for diseases caused by hypermyotonia such as strabismus, blepharospasm, facial spasms, spasmodic torticollis, paralysis after cerebral apoplexy, infantile cerebral paralysis, spasmodic phonopathy, headache such as migraine, chronic pain such as lumbago, stiff shoulder, muscular relaxation disorder accompanied with onset of Parkinson's disease or multiple sclerosis, myofascial pain syndrome, masticatory spasm, chronic anal fissure, urinary inconsistency, grinding of teeth, facial myokymia, tic, topical dystonia and wrinkles.

## Description

### Background Art

The present invention relates to therapeutic agents for diseases caused by hypermyotonia where an M toxin of type A botulinum toxin as an active ingredient and, more particularly, it relates to therapeutic agents for strabismus, blepharospasm, hemifacial spasms, spasmodic torticollis, paralysis after cerebral apoplexy, infantile cerebral paralysis, spasmodic phoropathy, headache, lumbago,neck pain, back pain, stiff shoulder, muscular relaxation disorder accompanied by Parkinson's disease or multiple sclerosis, myofascial pain syndrome, masticatory spasm, chronic anal fissure, urinary inconsistency, grinding teeth, facial myokymia, tic, topical dystonia, wrinkles, etc.

### Technical Field

*Clostridium botulinum* is an obligate anaerobic Gram-positive bacillus and it has been known that toxin produced by *Clostridium botulinum* has a high affinity to terminal area of peripheral nerve and causes botulism, where a main symptom is flaccid paralysis of whole-body skeletal muscle.

Toxin produced by *Clostridium botulinum* is classified into seven, A to G, according to the difference in antigenicity and may be further divided into M-, L- and LL toxins according to the difference in the structure of non-toxic protein bonding to neurotoxin. In JP-A-2003-9897, there is a description that, when a botulinum toxin solution containing M-, L- and LL toxins is passed through a lactose column, it is able to be separated into an M toxin (hemagglutinin-negative substance or HA-negative substance) showing no hemagglutinating activity and L- and LL toxins (hemagglutinin-positive substances or HA-positive substances) showing a hemagglutinating activity.

It has been known that, in type A botulinum toxin, there are three types, i.e. an M toxin (HA-negative substance) having molecular weight of about 300, 000 to which non-toxic component having no hemagglutinating activity to neurotoxin is bonded, an L toxin (HA-positive substance) having molecular weight of about 500,000 to which non-toxic component having hemagglutinating activity to an M toxin and an LL toxin (HA-positive substance) having molecular weight of about 900,000 where two L toxins are associated with each other.

Incidentally, strabismus is caused by a breakdown of a balance in strain among sphincter muscles and blepharospasm is a disease where opening of eye becomes difficult by involuntary contraction of orbicular muscles of the eye. Facial spasms is a disease where facial muscle involuntarily shrinks due to irritability accentuation of facial nerves while spasmodic torticollis is a disease where abnormality is caused in head due to abnormal tension of muscle of neck. Masticatory spasm is a disease where involuntary movement happens in the lower jaw and movement of pulling up the lower jaw in both sides (closing the mouth) happens involuntarily. Grinding of the teeth is a symptom like masticatory spasm and is a phenomenon during sleeping being thought to be a kind of sleep disorder. Spasmodic phonopathy is a disease where involuntary contraction happens in motor muscle of vocal cord whereby normal utterance is unable to be done. In facial myokymia, spasm of muscular fillets happens in a part of muscle bundle of the face in groups and, in the skin or the surface of mucous membrane, swaying or swelling sustained involuntary movement is observed. Tic is sometimes called Tourette's syndrome and is a disease where spasmodic, dramatic and sudden muscle spasm happens. Its examples are nictiation, wry face, head shaking and crying. Chronic anal fissure is accompanied by over-tension of anal sphincter caused by repetitive anal fissure and is a disease resulting in anal stenosis due to loss of elasticity of anus. In imminent urinary incontinence, a part of muscles of bladder strains excessively in an involuntary manner whereby a strong urination feeling is apt to be noted and it is a disease to urinate regardless of one's will. Myofascial pain syndrome is a disease where, as a result of acute disorder or repetitive overloaded stress (too much use) of muscle, hard stiffness-like part (strain belt) is formed in muscle and strong pain occurs and it has been known that muscle strain of hand and foot is excessively promoted after cerebral apoplexy or as a result of onset of infantile cerebral palsy, Parkinson's disease or multiple sclerosis. It has been also known that headache such as migraine occurs chronically when strain of muscles of neck or shoulder is excessively promoted and that strain of muscle is abnormally promoted by too much use of muscle or a sustained poor posture and, as a result, chronic pain or stiff neck such as lumbago, neck pain and back pain is induced. Wrinkles are generated by shrinking of muscle and examples of wrinkles on the face are wrinkles between the brow, wrinkles at corner of the eye and wrinkles at nose root. As such, all of those strabismus, blepharospasm, facial spasms, spasmodic torticollis, paralysis after cerebral apoplexy, infantile cerebral paralysis, spasmodic phonopathy, headache such as migraine, chronic pain such as lumbago, stiff neck, muscular relaxation disorder accompanied with onset of Parkinson's disease or multiple sclerosis, myofascial pain syndrome, masticatory spasm, chronic anal fissure, urinary inconsistency, grinding of teeth, facial myokymia, tic, topical dystonia and wrinkles are caused by hypermyotonic action.

With regard to utilization of botulinum toxin for treatment of diseases caused by hypermyotonia, its examples are that botulinum toxin is used for treatment of strabismus in *Ophthalmology,* 87, 1044-1049 (1980) and that, in *J*. *Fr. Ophthalmol.* 13, 259-264 (1990), it is used for treatment of blepharospasm. In JP-A-8-511536, there is disclosed a method where botulinum toxin is administered until a decrease in clinical response is caused and, after that, other botulinum toxin is administered to treat the disease of nerve muscle. In JP-A-8-511537, there is disclosed a method where at least two of botulinum toxins of type A to type G are jointly administered whereby the disease of nerve muscle is treated. In "Therapy with Botulinum Toxin", Marcel Dekker, New York, 1994, pages 577-595, it is reported that botulinum toxin is effective for treatment of wrinkles between brow.

Actually, as a botulinum toxin effective for the treatment of blepharospasm, one-side face spasm, spasmodic strabismus, wrinkles, etc., BOTOX (registered trademark) (manufactured by Allergan) where an LL toxin of botulinum toxin is an active ingredient has been sold in the market.

However, in any of the aforementioned literatures, there is neither description nor suggestion at all for application of an M toxin of type A botulinum toxin (HA-negative substance) for the treatment of diseases caused by hypermyotonia such as strabismus, blepharospasm and wrinkles.

### Disclosure of the Invention

Although botulinum toxin has been known as a drug for mitigation of muscle strain, the toxin itself is a drug having strong toxicity. Therefore, as a result of its side effect, botulinum toxin may cause systemic malaise by mitigation of muscle strain. Particularly when there is a mistake for its dose, severe side effect happens whereby it is desirable that dose of botulinum toxin is made as little as possible. In addition, a problem that, when botulinum toxin is repeatedly administered, its efficacy is attenuated has been pointed out and the phenomenon as such is thought to be dependent upon production of antibody to the toxin. Accordingly, a therapeutic agent which does not induce antibody production and does not lower its effect even when administered repeatedly has been demanded. Although BOTOX (registered trademark) where an LL toxin of botulinum toxin is an active ingredient has been sold in the market already, there has been a demand for far better products in view of pharmaceutical effect and side effect.

The present inventors have carried out intensive studies paying their attention to the constituting components (M toxin, L toxin and LL toxin) of type A botulinum toxin and, as a result, they have found that an M toxin (HA-negative substance) having molecular weight of about 300,000 being bonded to non-toxic component showing no agglutination activity to neurotoxin has a better inhibiting activity to nerve muscle transmission than a mixture of an L toxin having molecular weight of about 500, 000 being bonded to non-toxic component showing agglutination activity to an M toxin and an LL toxin having molecular weight of about 900,000 (HA-positive substance), has a therapeutic index of five times higher than commercially available BOTOX (registered trademark), rarely produces an antibody and is able to maintain its effect even upon repetitive administration whereby an M toxin of type A botulinum toxin is particularly effective as a therapeutic agent for various diseases caused by hypermyotonia whereby the present invention has been achieved.

The present invention relates to an inhibitor for neuromuscular transmission or a therapeutic agent for the treatment of diseases caused by hypermyotonia comprising an M toxin (HA-negative substance) of type A botulinum toxin as an active ingredient.

The M toxin of type A botulinum toxin according to the present invention has an excellent inhibiting activity for neuromuscular transmission and a high therapeutic index, rarely induces the antibody production and shows little attenuation of the effect even upon repetitive administration whereby it is useful as a therapeutic agent for diseases caused by hypermyotonia such as strabismus, blepharospasm, facial spasms, spasmodic torticollis, paralysis after cerebral apoplexy, infantile cerebral paralysis, spasmodic phonopathy, headache such as migraine, chronic pain such as lumbago, stiff shoulder, muscular relaxation disorder accompanied with onset of Parkinson's disease or multiple sclerosis, myofascial pain syndrome, masticatory spasm, chronic anal fissure, urinary inconsistency, grinding of teeth, facial myokymia, tic, topical dystonia and wrinkles. Examples of wrinkles are those on the face such as wrinkles between the brow caused by shrinking of facial muscle, wrinkles at corner of the eye, wrinkles at nose root and wrinkles at the chin.

Although details thereof will be mentioned under the item of pharmacological tests, an inhibitive activity for nerve transmission of an M toxin (HA-negative substance) of type A botulinum toxin was evaluated by conducting a test on muscular tension. In the muscular tension test, specimens of diaphragm and phrenic nerve of mice were used and, as a comparative control drug, a mixture of L- and LL toxins of type A botulinum toxin was used. By conducting a test on grip of hind paw of mice, therapeutic index (TD₂₀/ED₅₀) of an M toxin (HA-negative substance) of type A botulinum toxin was evaluated (here, ED₅₀ is a 50% effective dose while TD₂₀ is a 20% toxic dose) . Further, antigenicity of an M toxin of type A botulinum toxin was evaluated by a test for the measurement of antibody value. In the grip test of hind paw and measurement test for antibody value as such, mice were used and, as comparative control drugs, a mixture of L- and LL-toxins of type A botulinum toxin and a commercially available BOTOX (registered trademark) were used. Although those tests using comparative control drugs as such are standard in conducting in the same molar ratio, biological activity (titer) of the toxin is used as a standard because it is difficult to specify the molecular weight of a mixture of L- and LL toxins. Thus, titer of the toxin is able to be expressed by an i.p. LD₅₀ value (50% lethal dose by intraperitoneal administration) and the test was conducted at the dose where the i.p. LD₅₀ values were the same.

Although an M toxin having the same titer as that of a mixture of L- and LL toxins was used, it has been found as a result of muscular tension test that the M toxin achieves an inhibitory activity for nerve transmission about 10 times as high as that of a mixture of L- and LL toxins. It is usual to predict that toxins having the same titer achieve the same effect but the aforementioned finding is entirely contrary to such a prediction. As a result of a grip test of hind paw, it has been also found that the therapeutic index (TD₂₀/ED₅₀) of an M toxin of type A botulinum toxin is about five-fold that of the commercially available BOTOX (registered trademark). As a result of the test for measurement of antibody value, it has been further found that antigenicity of an M toxin of type A botulinum toxin is lower than that of BOTOX (registered trademark) and that an M toxin of type A botulinum toxin has less reduction in the effect even upon repeated administration as compared with BOTOX (registered trademark). From those results, it is likely that, when an M toxin of type A botulinum toxin is used, far higher therapeutic effect is achieved and occurrence of side effects such as systemic malaise is able to be effectively suppressed.

With regard to an M toxin of type A botulinum toxin according to the present invention, when incubation is carried out using a strain which does not produce an HA-positive toxin such as 7I03-H, 7I05-H, Chiba-H, Kyoto-F and 804-1H among the type A *Clostridium botulinum,* it is possible to produce only M toxin of type A botulinum toxin whereby an operation for the separation of L- and LL toxins can be omitted. It is also possible that, when a botulinum toxin solution containing M-, L- and LL toxins is passed through an ion-exchange column or a gel filtration column, only M toxin having no agglutination activity is separated from the mixture.

An M toxin of type A botulinum toxin does not have agglutination activity (HA-negative) and its molecular weight is within a range of 200,000 to 400,000. On the contrary, Land LL toxins of type A botulinum toxin have agglutination activity (HA-positive) and their molecular weights are 500,000 or higher. Accordingly, an M toxin of type A botulinum toxin is able to be clearly distinguished from L- and LL toxin thereof.

Dose of an M toxin of type A botulinum toxin according to the present invention is able to be appropriately determined depending upon the object diseases and there is no particular limitation therefor. When, however, side effect by toxin is taken into consideration, it is preferred to be 0.01 to 500 unit(s)/site per treatment and, more preferably, it is 0.5 to 300 unit(s)/site.

It is preferred that the therapeutic agent for diseases caused by hypermyotonia in accordance with the present invention is administered to muscle which is an acting site of the botulinum toxin. The dosage form thereof is mostly injection preparation and is able to be made into pharmaceutical preparation using a widely used art.

The injection preparation of the present invention is able to be prepared by addition of additives such as an osmotic pressure adjusting agent (e.g., sodium chloride) and a buffer (e.g., sodium phosphate).

It is preferred that pH of the injection preparation of the present invention is adjusted at 4.0 to 7.5 and it is preferred that an osmotic pressure ratio is adjusted at near 1.0.

The therapeutic agent for diseases caused by hypermyotonia according to the present invention can be injected by a commonly conducted intramuscular injection.

### Brief Description of the Drawing

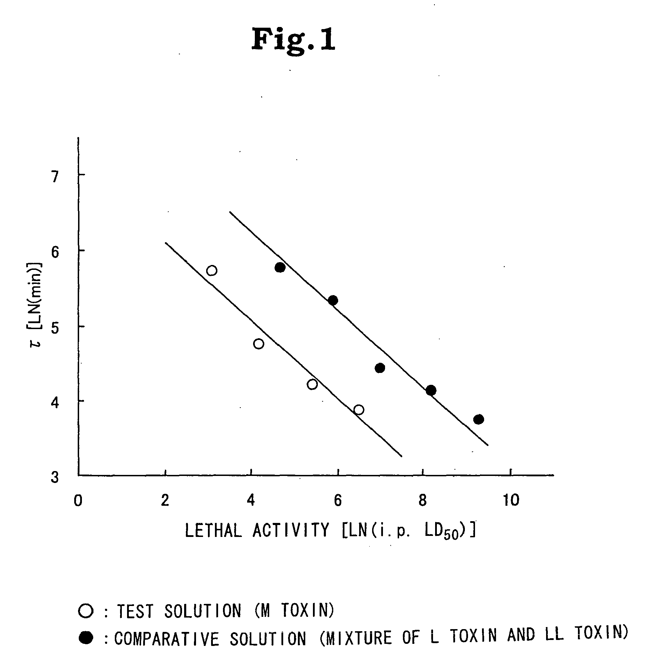

Fig. 1 is a graph which shows the relation between lethal activity and inhibitive activity for neuromuscular transmission in mice when a test solution and a comparative solution were used.

### Best Mode for Carrying Out the Invention

Production examples, pharmacological tests and preparation examples are described as hereunder and those examples are intended for better understanding of the present invention and do not limit the scope of the present invention.

### 1. Production of the toxin for the test

The toxin for the test of type A botulinum toxin was produced by a method of Sakaguchi (Sakaguchi, G. (1983): *Clostridium botulinum* toxins. *Pharmac. Ther.,* 19, 165-94) with partial modifications.

### (1) Production Example 1 (Production of toxin of an M toxin)

Spore cell solution of type A 7I03 strain (a strain which produces "M toxin") of *Clostridium botulinum* stored by freezing was inoculated to a pre-incubation medium (cooked meat medium) and incubated at 30°C for 2 days. The pre-incubation medium was planted to a peptone-yeast extract-glucose medium (PYG medium) and incubated at 30°C for 3 days.

Next, 3N H₂SO₄ solution was added to the culture solution to conduct a precipitation with an acid, the culture solution was adjusted to pH 3.5 and allowed to stand at room temperature for one night. On the next day, it was centrifuged (9200 × g, 20 minutes, 4°C) and the resulting precipitate was dissolved in 0.2M phosphate buffer (pH 6.0).

The dissolved solution was adjusted to pH 6. 0 and stirred at 37°C for 1 hour to extract the toxin. The extract was centrifuged (9,200 × g, 20 minutes, 4°C), its supernatant liquid was collected and 0.2M phosphate buffer (pH 6.0) containing 2% of protamine was added to give a precipitate. This was centrifuged (9,200 × g, 15 minutes, 4°C) to give a supernatant liquid. To this supernatant liquid was added a saturated ammonium sulfate solution (390 g/L) so as to make the sulfate 60% (w/v) and the mixture was allowed to stand for one night at 4°C to conduct a salting-out. On the next day, centrifugal separation (9,200 × g, 15 minutes, 4°C) was conducted, the obtained precipitate was dissolved in 0. 05M acetate buffer (pH 4.2, 0.2M NaCl) and the obtained solution was dialyzed with a permeable membrane against 0.05M acetate buffer (pH 4.2, 0. 2M NaCl) for one night. After completion of the dialysis, it was centrifuged (13,700 × g, 15 minutes, 4°C) and the obtained supernatant liquid was subjected to ion-exchange using a column [SP-Sepharose Fast Flow (Amersham)] equilibrated with 0.05M acetate buffer (pH 4.2, 0.2M NaCl). This was subjected to a linear gradient so as to give 0.05M acetate buffer (pH 4.2, 0.7M NaCl) finally and, as a result, impurities and toxin were eluted.

A toxin fraction was collected with a fraction collector and the portion which is to be collected as "M toxin" was determined on the basis of toxicity measurement, OD value and electrophoretic images. The collected fraction was concentrated by ultrafiltration (Amicon YM 30) and, in order to raise its purity, the concentrate was passed through a gel filtration column [Sephadex G-200 (Pharmacia)] equilibrated with 0.05M acetate buffer (pH 6.0, 0.2M NaCl) and the collected toxin fraction was concentrated by ultrafiltration (Amicon YM 30) to obtain an original liquid (protein concentration: 1.78 mg/ml; biological activity: 2.0 × 10⁷ i.p. LD₅₀/ml) of "M toxin" which is a type A botulinum toxin for a pharmacological test. Incidentally, with regard to the biological activity (titer), a 50% lethal dose (i.p. LD₅₀) after intraperitoneal administration to mice was used as an index.

### (2) Production Example 2 (Production of a mixture of L toxin and LL toxin)

The same operation as in Production Example 1 was carried out using *Clostridium botulinum* type A 62A strain (strain which produces "a mixture of M toxin, L toxin and LL toxin") instead of *Clostridium botulinum* A 7I03H strain to give an original liquid (protein concentration: 1.99 mg/ml; biological activity: 1.3 × 10⁷ i.p. LD₅₀/ml) of "a mixture of L toxin and LL toxin" which is a type A botulinum toxin for a pharmacological test. A toxin fraction was collected with a fraction collector and the portion which is to be collected as "a mixture of L toxin and LL toxin" was determined on the basis of toxicity measurement, OD value and electrophoretic images.

### 2. Pharmacological Test

### (1) Comparison of inhibitive activity for neuromuscular transmission based on muscular tension test of specimens of diaphragm and phrenic nerve of mice

In order to compare the pharmacological activities of "M toxin" and "a mixture of L toxin and LL toxin", a muscular tension test was carried out using diaphragm and phrenic nerve specimens of mice (strain: ddY; sex: male).

### (Preparation of test solution)

The M toxin of Production Example 1 was diluted with 20 mM Tris hydrochloride buffer containing 0.02% bovine serum albumin (pH 7.4; 150 mM NaCl) to prepare a test solution (3.4 × 10⁵ i.p. LD₅₀/ml).

### (Preparation of comparative solution)

A mixture of L toxin and LL toxin of Production Example 2 was diluted with 20 mM Tris hydrochloride buffer containing 0.02% bovine serum albumin (pH 7.4; 150 mM NaCl) to prepare a test solution (5.5 × 10⁵ i. p. LD₅₀/ml).

### (Measuring method)

Pentobarbital (50 mg/kg) was intraperitoneally administered to mice to euthanize them, the thorax was opened and right and left phrenic nerves were ligated at the height of thymus. Right and left phrenic nerves were carefully exfoliated from the surrounding connective tissue until diaphragm and then cutting was also done for abdominal side whereupon diaphragm and the twelfth rib were excised in a united manner where phrenic nerve was attached thereto. The excised diaphragm and phrenic nerve specimen was cut into one half in a water tank kept at 37°C and each half diaphragm piece was fixed to a tissue-supporting apparatus using a silk yarn being passed through the twelfth rib.

Next, the phrenic nerve was passed through a platinum electrode loop and transferred to a tissue bath where the diaphragm specimen was kept at 37°C. An end of silk yarn where another end thereof was ligated to diaphragm central tendon was connected to an isometric tension transducer and the diaphragm-phrenic nerve specimen was suspended in a Krebs solution. Rectangular wave where connecting time was 10 msec and voltage was 1 V was applied from a platinum electrode loop to phrenic nerve with a frequency of 0.25 Hz and shrinking tension of diaphragm induced by electric stimulation to the nerve was amplified with a tension amplifier and recorded with a pen recorder with a lapse of time. Load at a rest stage of about 4 g was applied to the diaphragm-phrenic nerve specimen and an observation was conducted every 15 to 20 minutes while exchanging the Krebs solution in the tissue bath for 1 to 2 hour(s) until induced tension and base line became stable without any treatment.

After confirming that base line and tension were stable, each of the test solution and the comparative solution was added to the Krebs solution in the tissue bath and attenuation of the tension induced by the toxin was recorded. After completion of the experiment, tension was analyzed for each of diaphragm and phrenic nerve specimens. As an index for attenuation of tension due to the action of the toxin, time from addition of the toxin until attenuation of the induced tension to an extent of 1/e of that immediately before the treatment with the toxin or, in other words, contraction tension which was recoded with elapse of time was plotted and an approximate line was determined whereby time (τ) for treating with toxin required for attenuation to an extent of 1/e of the contraction tension before treatment with toxin was determined and used as an index for inhibitive activity for neuromuscular transmission (incidentally, the smaller the τ value, the higher the neuromuscular transmission inhibiting activity). From those results, Fig. 1 shows the relation between lethal action, to mice and inhibitory activity of neuromuscular transmission when the test solution and the comparative solution were used and Table 1 shows the inhibitory activity of the comparative solution when the inhibitory activity for neuromuscular transmission of the test solution in the same lethal dose for mice was 1. Incidentally, each plot for the test solution in Fig. 1 is a mean value of 4 to 5 cases and each plot for the comparative solution therein is a mean value of 2 to 3 cases.

**Table 1**

| Inhibitory Activity for Neuromuscular Transmission | |
|---|---|
| Test Solution | 1.00 |
| Comparative Solution | 0.10 |

### (Result)

From Fig. 1 and Table 1, an inhibitory activity for neuromuscular transmission of the M toxin is about 10-fold that of a mixture of L toxin and LL toxin.

### (2) Comparative test for therapeutic index based on grip test of hind paw of mice

In order to compare the pharmaceutical effect (ED₅₀: 50% effective dose) and the toxicity (TD₂₀: 20% toxic dose) for "M toxin", "a mixture of L toxin and LL toxin" and "BOTOX (registered trademark) (manufactured by Allergan)", a grip test by hind paw of mice (strain: ddY; sex: male) was carried out.

### (i) Study of pharmaceutical effect (ED₅₀)

### (Preparation of test solutions)

The M toxin of Production Example 1 was diluted with a physiological saline containing 0.1% of human serum albumin to prepare five test solutions having different concentrations (1.2, 4, 12, 40 and 120 i.p. LD₅₀/ml).

### (Preparation of comparative solution A)

A mixture of the L toxin and LL toxin of Production Example 2 was diluted with a physiological saline containing 0.1% of human serum albumin to prepare five "comparative solution A"s having different concentrations (1.2, 4, 12, 40 and 120 i.p. LD₅₀/ml) in the same manner as in the test solutions.

### (Preparation of comparative solution B)

BOTOX (registered trademark) was diluted with a physiological saline containing 0.1% of human serum albumin to prepare five "comparative solution B"s having different concentrations (1.2, 4, 12, 40 and 120 i.p. LD₅₀/ml) in the same manner as in the test solution.

### (Measuring method)

To gastrocnemius of right hind paw of mice was administered each of solvent (a physiological saline containing 0.1% of human serum albumin), test solution, comparative solution A and comparative solution B at the dose of 250 µl/kg (dose in terms of toxin for test solution, comparative solution A and comparative solution B each was 0. 3, 1, 3, 10 and 30 i.p. LD₅₀/kg) . After 6 hours and 1, 2, 3, 7, 14 and 21 day(s) from the administration, grip of right hind paw was measured using a grip strength meter for small animals (ten animals per group). When the grip of the solvent administration group at each measuring time was defined 100, the grip after the administration of each toxin was determined. Using the value at the stage where lowering in the grip in each administration group was maximum, ED₅₀ values of test solution, comparative solution A and comparative solution B were calculated (Table 2).

### (ii) Study of toxicity (TD₂₀)

### (Preparation of test solution)

The M toxin of Production Example 1 was diluted with a physiological saline containing 0.1% of human serum albumin to prepare four test solutions having different concentrations (20, 50, 100 and 150 i.p. LD₅₀/ml).

### (Preparation of comparative solution A)

A mixture of the L toxin and LL toxin of Production Example 2 was diluted with a physiological saline containing 0.1% of human serum albumin to prepare four test solutions having different concentrations (20, 50, 100 and 150 i.p. LD₅₀/ml) in the same manner as in the test solutions.

### (Preparation of comparative solution B)

BOTOX (registered trademark) was diluted with a physiological saline containing 0.1% of human serum albumin to prepare four test solutions having different concentrations (20, 50, 100 and 150 i.p. LD₅₀/ml) in the same manner as in the test solutions.

### (Measuring method)

To quadriceps of right hind paw of mice was administered each of solvent, test solution, comparative solution A and comparative solution B at the dose of 500 µl/kg (dose in terms of toxin for test solution, comparative solution A and comparative solution B each was 10, 25, 50 and 75 i.p. LD₅₀/kg) . After 1, 2, 3, 7 and 14 day(s) from the administration, grip of left hind paw was measured using a grip strength meter (six to ten animals per group). This method measures the degree of muscle relaxation of the toxin in muscle of left hind paw which is another muscle whereto the toxin was leaked from quadriceps of right hind paw which is the administration site. When the grip of the solvent administration group at each measuring time was defined 100, the grip after the administration of each toxin was determined. Using the value at the stage where lowering in the grip in each administration group was maximum, TD₂₀ values of test solution, comparative solution A and comparative solution B were calculated (Table 2) .

### (iii) Therapeutic index (TD₂₀/ED₅₀)

Ratio of the ED₅₀ value for expression of pharmaceutical effect to the TD₂₀ value for expression of toxicity for each of test solution, comparative solution A and comparative solution B was calculated and the resulting ratio was defined as a therapeutic index. The therapeutic index is expressed as TD₂₀/ED₅₀. When the therapeutic index is higher, the deviating width between the dose showing the efficacy and the dose showing the toxicity is bigger. As a result, it means that a drug having high therapeutic index has a high usefulness as a drug. The results are shown in Table 2.

**Table 2**

| | ED₅₀ (i.p. LD₅₀/kg) | TD₂₀ (i.p. LD₅₀/kg) | Therapeutic Index |
|---|---|---|---|
| Test Solution | 0.57 | 37.3 | 65.4 |
| Comparative Solution A | 0.88 | 17.0 | 19.3 |
| Comparative Solution B | 1.27 | 16.2 | 12.8 |

### (Results)

It is apparent from Table 2 that the ED₅₀ value of the M toxin is less than that of a mixture of L toxin and LL toxin and that of BOTOX (registered trademark) whereby the M toxin has higher pharmaceutical effect than the mixture of L toxin and LL toxin and BOTOX (registered trademark) and that the TD₂₀ value of the M toxin is twice as bigger than those of a mixture of L toxin and LL toxin and BOTOX (registered trademark) whereby the M toxin has far lower toxicity than the mixture of L toxin and LL toxin and BOTOX (registered trademark). The therapeutic index of the M toxin (test solution) is 3.4-fold that of a mixture of L toxin and LL toxin (comparative solution A) and is 5.1-fold that of BOTOX (registered trademark) (comparative solution B).

### (3) Comparison of antigenicity based on the measurement test of antibody value

Comparison and study were carried out to check whether the antibody production was observed after repetitive administration of "M toxin", "a mixture of L toxin and LL toxin" and "BOTOX (registered trademark) (manufactured by Allergan)".

### (Preparation of test solution)

The M toxin of Production Example 1 was diluted with a physiological saline containing 0.1% of human serum albumin to prepare a test solution (100 i.p. LD₅₀/ml).

### (Preparation of comparative solution A)

A mixture of the L toxin and LL toxin of Production Example 2 was diluted with a physiological saline containing 0.1% of human serum albumin to prepare a comparative solution A (100 i.p. LD₅₀/ml) in the same manner as in the test solution.

### (Preparation of comparative solutions B)

BOTOX (registered trademark) was diluted with a physiological saline containing 0.1% of human serum albumin to prepare a comparative solution B (100 i.p. LD₅₀/ml) in the same manner as in the test solution.

### (Measuring method)

To quadriceps of right hind paw of mice was administered each of solvent (physiological saline containing 0.1% of human serum albumin), test solution, comparative solution A and comparative solution B at the dose of 500 µl/kg (dose in terms of toxin for test solution, comparative solution A and comparative solution B each was 25 i.p. LD₅₀/kg) at the rate of every three weeks and three times in total. After 14 days or 15 days from the third administration, plasma was collected from the mice (13 to 19 animals per group) and combined with the plasma collected from orbital vein before administration of the toxin and the obtained mixture was subjected to a measurement of antibody value by an ELISA method. Antibody was considered to be produced in the mouse where the absorbance ratio of the plasma collected after administration of toxin to that collected before the administration was 10 or more and the rate of the mice producing the antibody was calculated (Table 3).

**Table 3**

| | Rate of Mice wherein Antibody was Produced (%) |
|---|---|
| Test Solution | 16 |
| Comparative Solution A | 29 |
| Comparative Solution B | 31 |

### (Result)

As shown in Table 3, the rate of mice wherein antibody was produced in the group to which the M toxin was administered was about one half of the rate in the group to which a mixture of L toxin and LL toxin was administered or in the group to which BOTOX (registered trademark) was administered. Accordingly, antigenicity of the M toxin was lower than that of L toxin, LL toxin or toxin contained in BOTOX (registered trademark).

### (4) Comparison of attenuating rate of the effect by repetitive administration

In a grip test by hind paw of mice, it was studied whether the effect was attenuated by repetitive administration of "M toxin", "a mixture of L toxin and LL toxin" and "BOTOX (registered trademark) (manufactured by Allergan)".

### (Preparation of test solution)

The M toxin of Production Example 1 was diluted with a physiological saline containing 0.1% of human serum albumin to prepare a test solution (2 i.p. LD₅₀/ml).

### (Preparation of comparative solution A)

A mixture of the L toxin and the LL toxin of Production Example 2 was diluted with a physiological saline containing 0.1% of human serum albumin to prepare a comparative solution A (2 i.p. LD₅₀/ml) in the same manner as in the test solution.

### (Preparation of comparative solutions B)

BOTOX (registered trademark) was diluted with a physiological saline containing 0.1% of human serum albumin to prepare a comparative solution B (2 i.p. LD₅₀/ml) in the same manner as in the test solution.

### (Measuring method)

Gastrocnemius of right hind paw of mice was administered each of solvent (a physiological saline containing 0.1% of human serum albumin), test solution, comparative solution A and comparative solution B at the dose of 7.5 µl (dose in terms of toxin for test solution, comparative solution A and comparative solution B each was 0.015 i.p. LD₅₀) . After 1, 2 and 3 day(s) from the administration, grip of right hind paw was measured using a grip strength meter for small animals. After 28 days from the first administration, the second administration was carried out (administration method and dose were the same as those in the first time) and grip of right hind paw was measured after 1, 2 and 3 day(s) from the administration in the same manner as in the first administration (11 or 12 animals per group). When the grip of the solvent administration group at each measuring time was defined 100, the grip after the administration of each toxin was determined. With regard to each of them at the first and the second administrations, total (1 + 2 + 3 day(s) after administration) of the three measured day for each mouse was calculated. After that, mean value of the difference between the value at the first administration and that at the second administration was calculated for each total value whereupon an attenuating rate for the effect was calculated (Table 4). Incidentally, when the value was bigger, the effect at the second administration was more attenuated.

**Table 4**

| | Attenuating Rate (%) of Effect |
|---|---|
| Test Solution | 5 |
| Comparative Solution A | 48 |
| Comparative Solution B | 26 |

### (Result)

As will be apparent from Table 4, as compared with the attenuating rate of effect at the second administration of the M toxin, the attenuating rate of effect of a mixture of L toxin and LL toxin was about 10-fold and that of BOTOX (registered trademark) was about 5-fold. From those results, it is apparent that the M toxin hardly causes attenuation of the effect upon repetitive administration as compared with L toxin, LL toxin and BOTOX (registered trademark).

### 3. Example of pharmaceutical preparation

### Injection preparation

A common example, of pharmaceutical preparation of an injection preparation according to the present invention is as follows.

| Formulation 1 (in 100 mL) | |
|---|---|
| M toxin of type A botulinum toxin | 1,000 units |
| Human serum albumin | 75 mg |
| Physiological saline | q. s. |

### Industrial Applicability

As apparent from the result of the pharmacological test, an M toxin of type A botulinum toxin (HA-negative substance) has higher inhibitory action for neuromuscular transmission than a mixture of L toxin and LL toxin (HA-positive substance) and also has a therapeutic index of 3- to 5-fold that of a mixture of L toxin and LL toxin (HA-positive substance) or a commercially available BOTOX (registered trademark). Moreover, it hardly induces antigen production and, even when administered repeatedly, its attenuation in the effect is small and, accordingly, it is particularly useful as a therapeutic agent for diseases caused by hypermyotonia such as strabismus, blepharospasm, facial spasms, spasmodic torticollis, paralysis after cerebral apoplexy, infantile cerebral paralysis, spasmodic phonopathy, headache such as migraine, chronic pain such as lumbago, stiff shoulder, muscular relaxation disorder accompanied with onset of Parkinson's disease or multiple sclerosis, myofascial pain syndrome, masticatory spasm, chronic anal fissure, urinary inconsistency, grinding of teeth, facial myokymia, tic, topical dystonia and wrinkles.

## Claims

1. An inhibitor for neuromuscular transmission comprising an M toxin (HA-negative substance) of type A botulinum toxin as an active ingredient.

2. A therapeutic agent for a disease caused by hypermyotonia comprising an M toxin (HA-negative substance) of type A botulinum toxin as an active ingredient.

3. The therapeutic agent according to claim 2, wherein the disease caused by hypermyotonia is strabismus, blepharospasm, hemifacial spasms, spasmodic torticollis, paralysis after cerebral apoplexy, infantile cerebral paralysis, spasmodic phonopathy, headache, lumbago, neck pain, back pain, stiff shoulder, muscular relaxation disorder accompanied by Parkinson's disease or multiple sclerosis, myofascial pain syndrome, masticatory spasm, chronic anal fissure, urinary inconsistency, grinding of teeth, facial myokymia, tic, topical dystonia or wrinkles.

4. The therapeutic agent according to claim 2, wherein molecular weight of the M toxin of type A botulinum toxin is 200,000 to 400,000.

5. The therapeutic agent according to claim 2, wherein the M toxin of type A botulinum toxin is produced by a strain of *Clostridium botulinum* type A 7103-H, *Clostridium botulinum* type A Chiba or *Clostridium botulinum* type A Kyoto-F.

6. The therapeutic agent according to any of claims 2 to 5, wherein the dosage form is an injection preparation.
